(19) Europäisches Patentamt / European Patent Office / Office européen des brevets

(11) EP 2 932 247 B1

(12) **EUROPEAN PATENT SPECIFICATION**

(45) Date of publication and mention of the grant of the patent:
**03.05.2023 Bulletin 2023/18**

(21) Application number: **12806024.1**

(22) Date of filing: **17.12.2012**

(51) International Patent Classification (IPC):
**G01N 27/22** (2006.01)     **G01N 33/28** (2006.01)
**G01M 13/04** (2019.01)

(52) Cooperative Patent Classification (CPC):
**G01N 27/221; G01M 13/04; G01N 27/226; G01N 33/2888**

(86) International application number:
**PCT/EP2012/075714**

(87) International publication number:
**WO 2014/094813 (26.06.2014 Gazette 2014/26)**

(54) **MATRIX SENSOR FOR LUBRICATING GREASE CONDITION MONITORING**

MATRIXSENSOR ZUR ÜBERWACHUNG DES ZUSTANDS VON SCHMIERFETT

CAPTEUR MATRICIEL POUR LA SURVEILLANCE DE LA CONDITION D'UNE GRAISSE LUBRIFIANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.10.2015 Bulletin 2015/43**

(73) Proprietor: **Aktiebolaget SKF**
**415 50 Göteborg (SE)**

(72) Inventors:
• **LUGT, Piet**
  **NL-4132 BB Vianen (NL)**
• **LANG, Defeng**
  **NL-2614 HP Delft (NL)**
• **STORKEN, Jozef Maria**
  **NL-3435 ZN Nieuwegein (NL)**
• **PALLISTER, Dave M.**
  **Highland, Michigan 48357 (US)**

(74) Representative: **Kohl, Thomas et al**
**SKF GmbH**
**Gunnar-Wester-Strasse 12**
**97421 Schweinfurt (DE)**

(56) References cited:
CN-A- 102 353 703     US-A1- 2009 216 471
US-A1- 2011 125 475     US-B2- 7 043 402

• **F.J. DICKIN ET AL: "Determination of composition and motion of multicomponent mixtures in process vessels using electrical impedance tomography-I. Principles and process engineering applications", CHEMICAL ENGINEERING SCIENCE, vol. 48, no. 10, 1 January 1993 (1993-01-01), pages 1883-1897, XP055053385, ISSN: 0009-2509, DOI: 10.1016/0009-2509(93)80358-W**
• **YANG DAOYE,GUO RUI,WANG XIAORONG,XU CHUANLONG,WANG SHIMIN: "Application of electrical capacitance tomography on lubricating oil film in journal bearings", PROCEEDINGS OF THE CHINESE SOCIETY OF ELECTRICAL ENGINEERING, vol. 32, no. 5, 15 February 2012 (2012-02-15), pages 99-104, XP008160143, ISSN: 0258-8013**
• **Anonymous: "SKF Lubrication Products and Systems", , 31 October 2009 (2009-10-31), pages 4-19, XP055457864, Retrieved from the Internet: URL:http://www.tehimpex.si/pdf/lepila_maziva/SKF_MASTI.pdf [retrieved on 2018-03-08]**

• RYOSUKE MATSUZAKI ET AL: "Full-field monitoring of resin flow using an area-sensor array in a VaRTM process", COMPOSITES PART A: APPLIED SCIENCE AND MANUFACTURING, ELSEVIER, AMSTERDAM, NL, vol. 42, no. 5, 24 January 2011 (2011-01-24), pages 550-559, XP028177381, ISSN: 1359-835X, DOI: 10.1016/J.COMPOSITESA.2011.01.014 [retrieved on 2011-01-28]

**Description**

BACKGROUND OF THE INVENTION

1. Field of the Invention

The present invention relates to grease condition monitoring and in particular, to a matrix based capacitive sensor for detecting the level of water or base oil in grease or like substances. The invention also relates to the use of such a sensor for monitoring the presence of water and oil in grease and in particular to a method which allows detection of changes to grease condition in-situ.

2. Description of the Related Art

Reliable operation of mechanical systems is highly dependent on correct lubrication. Particularly for systems designed to operate for long periods with little maintenance, the condition of the lubricant is a key factor. The presence of water, particulates or other contaminants can lead to wear and damage of moving parts and even catastrophic failure of the entire mechanical system. Various systems have been implemented for monitoring the condition of oil in circulating lubrication systems. These systems frequently rely on circulation of oil past the sensor and may additionally be provided with filters for removing contaminants.

For non circulating systems, monitoring is more difficult. One system disclosed in CN102353703 uses an electrical capacitance tomography technique (Electrical Capacitance Tomography, called ECT) to determine oil condition within the gap of a plain bearing. Closed systems such as bearings are generally filled with an appropriate grease, maintained in position by seals which also protect the grease from ingress of contaminants. Water ingress decreases the lubricating ability of the grease and increases the corrosion damage on the bearing elements. Grease generally comprises a thickener such as a soap or fatty acid, which carries a quantity of lower viscosity base oil that facilitates lubrication. During use of the system, the base oil may gradually separate from the grease and escape from the system e.g. through seals. The remaining grease life can be determined by measuring the remaining oil percentage in the grease. US2011/125475 discloses the use of capacitance measurements to determine when metal to metal contact takes place within a bearing.

Capacitive sensors have been proposed for condition monitoring of grease by applying an alternating voltage. Nevertheless, existing systems appear sensitive to grease build-up and uneven distribution thereof. It would therefore be desirable to provide for in-situ determination of grease condition, in particular for distinguishing between water ingress and decrease in oil content. It would also be desirable to be able to identify other qualities of the grease, including measurement of a state of oxidation, corrosion damage and the like.

BRIEF SUMMARY OF THE INVENTION

According to the invention there is provided a grease condition monitoring sensor comprising: a substrate for engagement with the grease to be monitored; a two-dimensional matrix of electrodes distributed on the substrate, the electrodes being insulated from each other and arranged for contact with the grease, such that the grease acts as a dielectric between the electrodes; and an interrogation circuit for interrogating pairs of adjacent electrodes by application of an alternating voltage between the electrodes, measuring the current response and determining a capacitance of the respective equivalent circuits; and evaluating a composition of the grease based on the determined capacitance. By using a plurality of electrodes and interrogating them individually in pairs, greater accuracy and flexibility may be achieved in the analysis of the results. This is especially important in the context of mechanical systems such as bearings, where the grease may not be uniformly distributed. By determining the respective capacitances, information about the condition of the grease may be obtained.

In a preferred embodiment, the sensor may comprise at least eight electrodes, namely at least four electrode pairs in a simple configuration in which the interrogation circuit is connected to interrogate electrodes as either positive or negative electrodes. In this sense, it will be understood that for an AC only circuit neither of the electrodes will actually be either positive or negative but the number of combinations is limited to certain pairings. The sensor may however comprise more than 50 electrodes and preferably more than 100 electrodes.

Alternatively, the interrogation circuit may be connected to interrogate electrodes both as positive and as negative electrodes. In that case, as many as 28 combinations of electrode pairs may be made for the case of just eight electrodes if all possible pairings are considered.

In a further embodiment of the invention, the substrate may be provided to vibrate at a frequency for the prevention of grease build-up on the electrodes. Such vibration may be ultrasonic and may also be of fixed or variable frequency. Preferably, vibration may be achieved using piezoelectric elements provided on the substrate. In one embodiment, the substrate may comprise an integrated circuit element having piezoelectric transducers and electrodes and may even include portions of the interrogation circuit. Alternative means for preventing build up of grease may be considered, including heating elements for melting the grease. For this, the electrodes themselves may be used.

Most preferably, the interrogation circuit comprises: a multi-frequency alternating voltage source; a control circuit for applying a multi-frequency alternating voltage from the multi-frequency alternating voltage source to pairs of adjacent electrodes; a signal analyser arranged to analyse the current response to the applied voltages for determining a complex impedance of each equivalent circuit; a memory comprising preset values representative of the expected complex impedance for different conditions of the grease; and a processor for comparing the complex impedance with the preset values. As will be understood by the skilled person, the electrodes and the grease together may be represented by an equivalent circuit comprising capacitance and resistance elements. By measuring not only the capacitance but also the resistance of this equivalent circuit, characteristics of the grease can be determined and compared with preset values that may be stored in an appropriate memory. The preset values may be provided in the form of look-up tables, generated in advance for the specific grease contained within the system. It is also conceivable that the preset value may be generated in real time from e.g. a known sample of grease. Evaluating the results at more than one frequency allows specific identification of the grease condition in that it is possible to distinguish between the presence of water and a reduction in base oil. Both of these conditions can lead to an increase in capacitance and a reduction in resistance but the relative changes are different at different frequencies.

Preferably, the interrogation circuit or its processor chooses a plurality of electrodes based on the current response of their equivalent circuits and evaluates the grease condition on the basis of the plurality of electrodes. This may involve selecting a plurality of electrodes that is less than the totality of the electrodes in the matrix. Such a selection allows evaluation of the grease condition only on the basis of electrodes indicating reliable results, e.g. those electrodes that clearly indicate that grease is present rather than air. Depending on the extent of the matrix and the number of electrodes needed for a reliable result, less than half of the electrodes may actually be used. In some case, less than 20% of the electrodes may be used or required to provide accurate result.

According to a further aspect of the invention, the interrogation circuit may comprise a microcircuit in direct electrical contact with the electrodes. The microcircuit may comprise its own source of power. The memory and the processor may form part of the same microcircuit. Alternatively, the processor may be located remotely from the electrodes and the system may comprise a transmission arrangement for transmitting data representative of the current response from the electrodes to the processor. The data may also be logged for periodic readout.

According to an important aspect of the invention, the substrate is preferably made of insulating material. It is however not excluded that the substrate may be conducting and may form one electrode, with the matrix electrodes being insulated from the substrate. In a further preferred embodiment, the substrate may be provided as part of a seal in a mechanical system. In one preferred embodiment the system is a sealed bearing. The substrate may be flexible, e.g. of plastics material.

The invention further relates to a method of in-situ condition monitoring of grease within a mechanical system, the method comprising: providing a matrix of electrodes separated from each other and at least partially in contact with the grease, such that the grease acts as a dielectric between at least some of the electrodes; interrogating pairs of adjacent electrodes by application of an alternating voltage between the electrodes, measuring the current response and determining the capacitance of the equivalent circuits; and evaluating a composition of the grease based on the determined capacitance. The method may use the sensor as described above.

Most preferably, evaluating the composition of the grease may comprise comparing the capacitances with preset values to determine whether the current response is indicative of the presence of grease between the respective electrode pairs; selecting electrode pairs having a current response indicative of the presence of grease between the electrodes and evaluating the composition of the grease based on the determined capacitance of the selected electrode pairs. In this manner, as indicated above, the method may be optimised to use those electrode pairs that are best suited.

The method may further comprise selecting electrode pairs that are contiguous to one another and define a single sensing region. The interrogation of the electrodes may lead to an effective mapping of the matrix into regions having grease and other regions in which grease is absent or sparse. The skilled person will be aware of suitable algorithms for mapping matrix locations based on a signal associated with each location. Such mapping algorithms may be similar to those used for pixel evaluation in a digital imaging system.

In a further preferred embodiment of the method, interrogation of the electrode pairs comprises application of a multi-frequency alternating voltage and furthermore determining of the complex impedance of the equivalent circuit. The alternating voltage may be applied over a spectrum of frequencies and the complex impedance can be evaluated over the spectrum. In this manner, the method may be used to compare the complex impedance with preset values representative of water content of the grease. An actual water content of the grease may then be determined based on a comparison with preset values for different water contents. Alternatively, a single value may be used indicative of an alarm condition and the method may comprise issuing an alarm when the preset value is exceeded.

The method may also be used to compare the complex impedance with preset values representative of oil content of the grease. In this manner, the actual amount of remaining base oil in the grease, or at least an indication thereof, may be determined. Based on these values, a decision may be taken to e.g. stop, service or replace the mechanical system.

Preferably, the alternating voltage is a low voltage source. In particular, the rms voltage may be less than 2 V, preferably less than 1 V and most preferably without a DC offset. A voltage applied to the grease may lead to electrolysis or decomposition thereof and can also be detrimental to the electrodes. For this reason, a low voltage is preferred subject to adequate signal strength.

According to a further aspect of the invention, the alternating voltage is preferably applied over a frequency range from 0.1 Hz to 1 MHz, preferably from 1 KHz to 1 MHz. It may also be desirable to analyse the signal of specific frequency ranges particular to the oil and to water. In particular, operation at resonant frequencies of these substances may provide greater sensitivity to their detection. The voltage may be applied at a number of discrete frequencies or over a spectrum of frequencies. The complex impedance may be evaluated at individual frequencies or over the full spectrum.

BRIEF DESCRIPTION OF THE DRAWINGS

The features and advantages of the invention will be further appreciated upon reference to the following drawings of a number of exemplary embodiments, in which:

Figure 1 shows an equivalent circuit on which measurements according to the invention are based;

Figure 2 shows an experimental setup for estimating the complex impedance of a grease sample;

Figure 3 shows a graph of water in grease capacitance response as a function of frequency for various water contents;

Figure 4 shows a graph of capacitance measurements for water in grease as a function of water percentages for different frequencies;

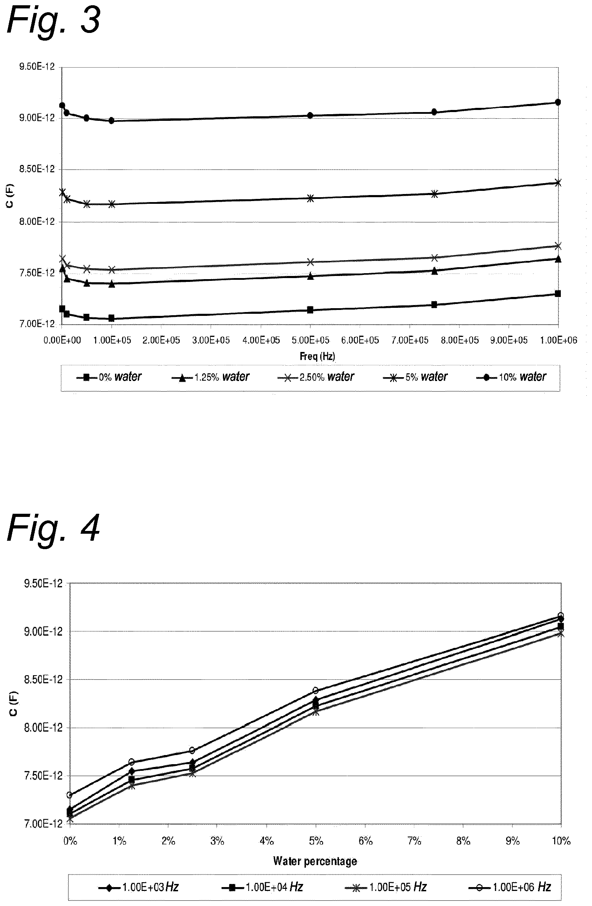

Figure 4 shows a graph of capacitance measurements for water in grease as a function of water percentages for different frequencies;

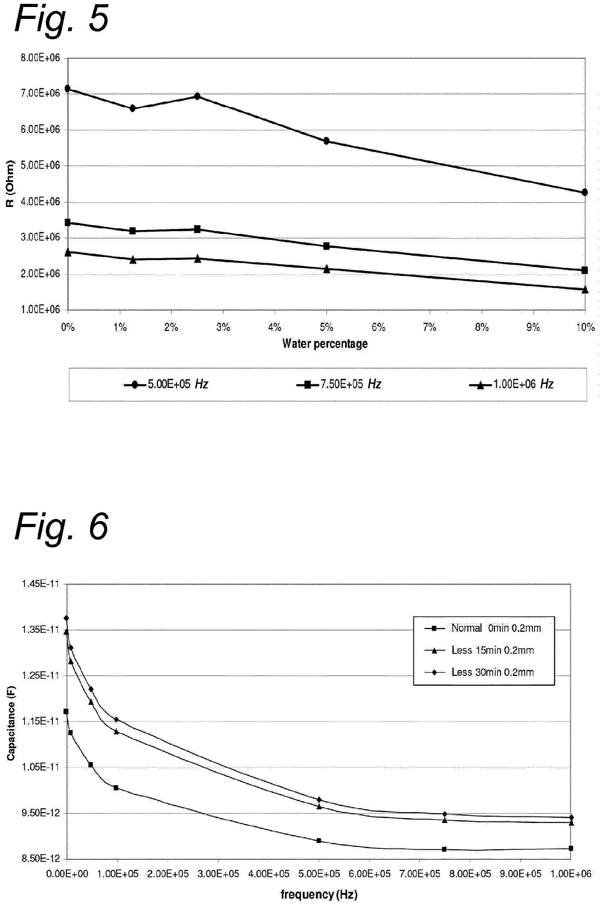

Figure 5 shows a graph of resistance measurements for water in grease as a function of water percentages for different frequencies;

Figure 6 shows a graph of capacitance measurements for oil in grease as a function of frequency for different levels of oil removal;

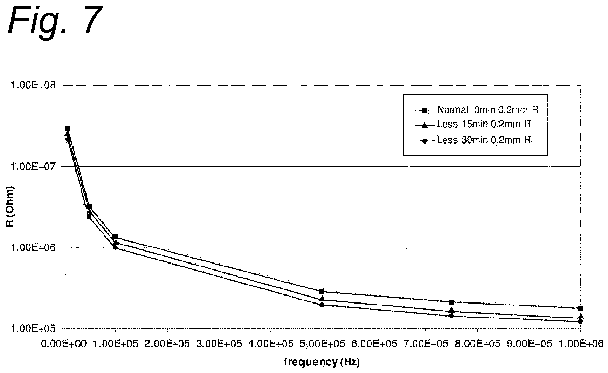

Figure 7 shows a graph of resistance measurements for oil in grease as a function of frequency for different levels of oil removal;

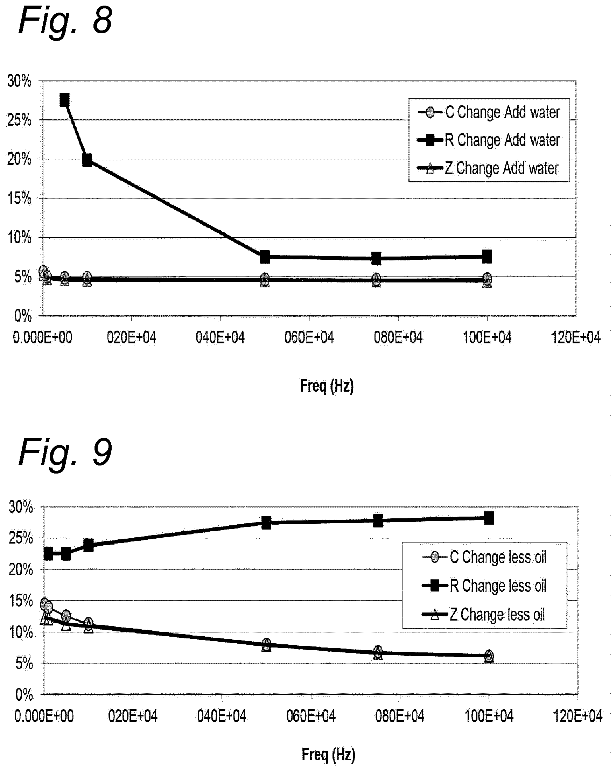

Figure 8 shows the parameter sensitivity of capacitance, resistance and impedance as a function of frequency for the case of 1.25 % water content;

Figure 9 shows the parameter sensitivity of capacitance, resistance and impedance as a function of frequency for the case of reduced oil content;

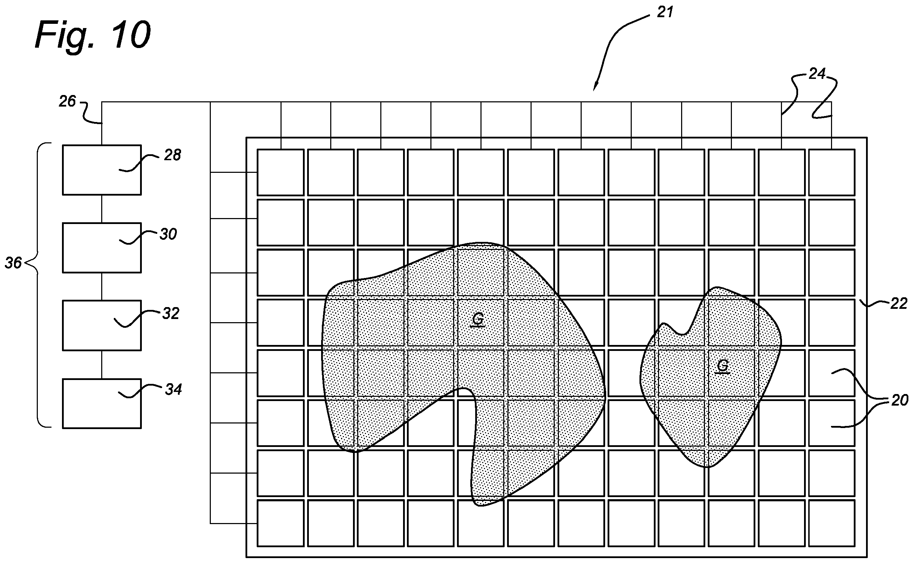

Figure 10 illustrates a matrix sensor according to the invention; and

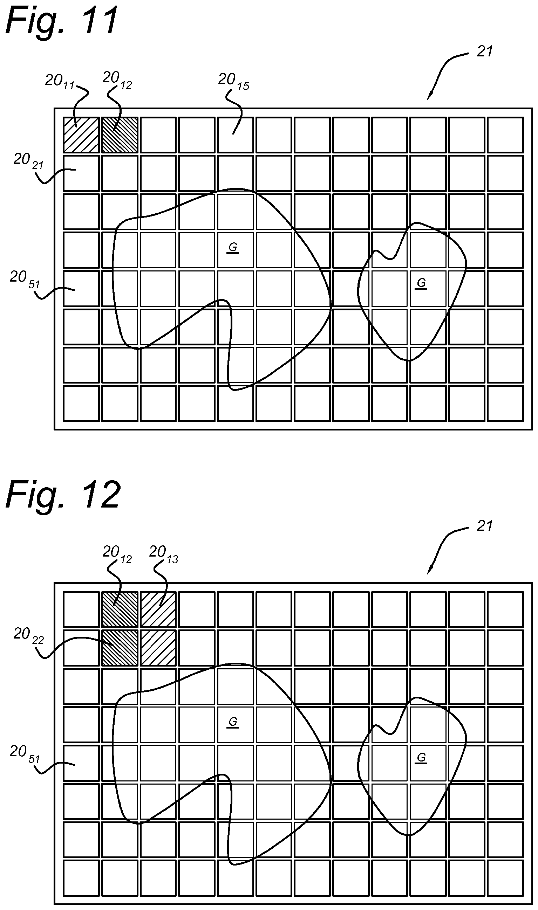

Figures 11 to 14 illustrate different electrodes being interrogated to detect the condition of the grease.

DESCRIPTION OF ILLUSTRATIVE EMBODIMENTS

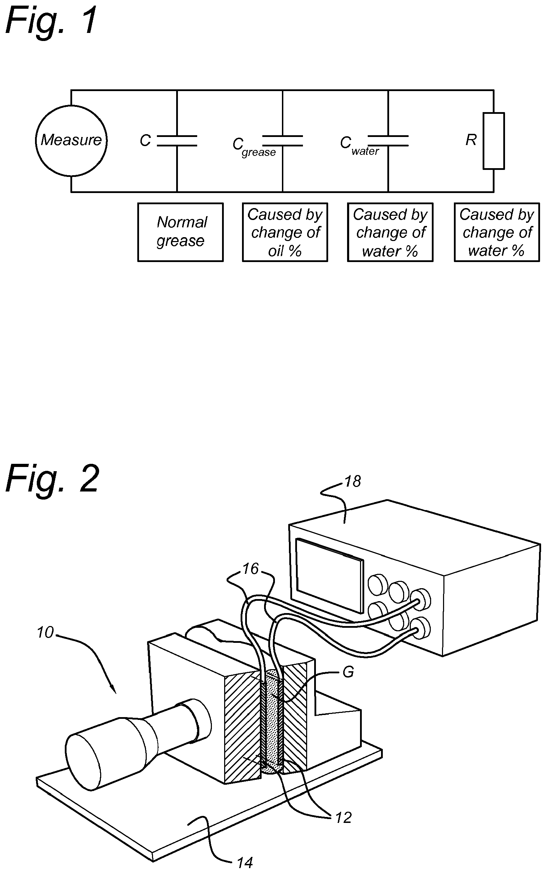

The general principle of operation of capacitive and complex impedance based sensors will first be explained with reference to Figures 1 to 9.

When an electric potential is applied via a pair of electrodes to a material or composition sample, the complex impedance of the sample can be measured. Due to the different electrochemical behaviour of different materials or compositions, unique impedance responses can be identified. By calibrating the spectroscopic impedance pattern for a given grease to the presence of water and loss of base oil, the occurrence of such conditions in sity can be identified.

For a parallel plate capacitor, its capacitance can be described by the dimensions of the plate and the permittivity of the material between the plates, written in Eq. (1).

$$C = \varepsilon_r \varepsilon_0 \frac{A}{d} \tag{1}$$

where

C is the capacitance;

A is the area of overlap of the two plates;

$\varepsilon_r$ is the relative static permittivity of the material between the plates (for a vacuum, $\varepsilon_r = 1$);

$\varepsilon_0$ is the electric constant ($\varepsilon_0 \sim 8.854 \times 10\text{-}12$ Fm-1); and

d is the separation between the plates.

Lubricating grease consists of thickener and base oil. Both oil and thickener have high resistivities and can be approximated as capacitors. The change of oil percentage will change the overall permittivity of the grease, which results in a different capacity of the system. When water with contaminations, e.g. tap water, is added to grease, the capacitance of the system changes due to the permittivity difference between water and grease. Meanwhile the resistivity of the system drops since tap water causes current leakage in the circuit. The capacitance change due to adding certain percentage (x %) of water can be written as Eq. (2).

$$\begin{aligned} C &= (\varepsilon_{water} \cdot x\% + \varepsilon_{grease} \cdot (1 - x\%))\varepsilon_0 \frac{A}{d} \\ &= \varepsilon_{water}\varepsilon_0 \cdot x\% \cdot \frac{A}{d} + \varepsilon_{grease}\varepsilon_0 \cdot (1 - x\%) \cdot \frac{A}{d} \\ &= C_{water} + C_{grease} \end{aligned} \tag{2}$$

Where:

C is the capacitance;
$\varepsilon_{water}$ is the relative permittivity of water between the plates ($\varepsilon_{water} \sim 80$);
$\varepsilon_{grease}$ is the relative permittivity of grease between the plates ($\varepsilon_{grease} \sim 2.4$);
$C_{water}$ is the capacitance of water; and
$C_{grease}$ is the capacitance of grease.

It can be seen from Eq. (2) that when water is added to grease, the initial capacitor C (with only grease) is split into $C_{water}$ and $C_{grease}$, with the $C_{water}$ and $C_{grease}$ connected in parallel. If the change in A or d due to the split of the capacitor are neglected, increasing water in grease will be equivalent to adding a capacitor $C_{water}$ in parallel to $C_{grease}$ in the measurement circuit. Since water with ion contaminations is conductive, a resistance is added to the circuit. The electrical model of this base oil, thickener, water system is shown in Figure 1.

Example 1

An experimental setup 10 is shown in Figure 2. The experimental setup comprised a parallel plate capacitor construction, in which the separation distance between two metal plates 12 was adjustable. The two plates 12 measured 10 mm × 10 mm each and were mounted in a micrometer 14, directly facing each other. The separation distance could be set from 0 mm to 5 mm with a resolution of 10 micron (0.01 mm) and accuracy of approximately 3 micron. A sample of grease G was placed between the plates 12, ensuring full coverage of both surfaces.
The plates 12 were electrically connected by leads 16 to a programmable automatic RCL meter (sometimes known as LCR meter) 18. The device was a FLUKE PM6306™, equipment number 0501 available from Fluke Corporation. The RCL meter 18 measures the output voltage (V), current (A) and phase difference for a given voltage and frequency input setting. The resistance, impedance and capacitance of the target system can then be calculated. The detailed description of the calculation method is not further described here, being generally conventional and otherwise according to the user manual of the RCL meter.

Samples: Two types of grease samples were used, one for the water in grease test, the other for the oil in grease test. The grease samples used in the water in grease test were SFK GJN, with water percentages of 0%, 1.25%, 2.5%, 5%, and 10% measured in weight. For the oil in grease test, samples of MT33 were used. Samples with different oil content were prepared by putting grease in a centrifuge for 15 minutes and 30 minutes respectively. The experimental setup is shown in Figure 2.

AC voltage input was used for the measurements. In order to reduce electrochemical reaction at the interface between the grease and the plates, the AC input voltage was set to 1 V, which is found sufficiently high for sensing purposes. The DC component of the input was also set to zero to minimize electrolysis on the interface, which otherwise could damage the repeatability for instant measurement or lower stability in a continuous test. Seven frequencies, 1 KHz, 10 KHz, 50 KHz, 100 KHz, 500 KHz, 750 KHz, and 1 MHz, were selected for investigation.

On the RCL meter 18, the circuit selection for the measurement was set to AUTO. At this setting, the device can detect and evaluate whether the target system behaves in a more capacitive or more resistive manner and whether it is more parallel or serially connected. This information is useful for verifying the accuracy of the equivalent circuit. As will be shown later, the water and grease composition indeed appeared as a circuit of parallel capacitors and resistors.

Two separation distances, 0.2 mm and 0.3 mm were used for all tests. For each sample, tests were started with a 0.3 mm gap setting for data acquisition over all 7 frequencies. Next, the test was repeated with the 0.2 mm gap without changing the sample. Before and after testing of each sample the parallel plates were cleaned. The surfaces of the plates were visually inspected for the possible occurrence of corrosion or damage.

The measurement results of the water in grease test with the 0.2 mm gap are shown in Table 1. The phase measurement results showed an angle between -90 to -89 degree. This indicates that the system behaves as a capacitive circuit.

Table 1

| Water | Gap (mm) | FREQ (Hz) | V(V) | I(A) | p(degree) | Q | C(F) | R (0hm) | (0hm) |
|---|---|---|---|---|---|---|---|---|---|
| 0% | 0.2 mm | 1.00E+03 | 1 | 4.49E-08 | -89.7 | 207 | 7.15E-12 | 1.00E+12 | 2.23E+07 |
| | 0.2 mm | 1.00E+04 | 1 | 4.46E-07 | -89.8 | 265 | 7.10E-12 | 1.00E+12 | 2.24E+06 |
| | 0.2 mm | 5.00E+04 | 1 | 2.22E-06 | -89.8 | 278 | 7.07E-12 | 1.25E+08 | 4.50E+05 |
| | 0.2 mm | 1.00E+05 | 1 | 4.44E-06 | -89.8 | 313 | 7.06E-12 | 7.05E+07 | 2.2.5E+05 |
| | 0.2 mm | 5.00E+05 | 1 | 2.24E-05 | -89.6 | 160 | 7.14E-12 | 7.14E+06 | 4.46E+04 |
| | 0.2 mm | 7.50E+05 | 1 | 3.39E-05 | -89.5 | 116.2 | 7.19E-12 | 3.43E+06 | 2.95E+04 |
| | 0.2 mm | 1.00E+06 | 1 | 4.59E-05 | -89.5 | 119.3 | 7.30E-12 | 2.60E+06 | 2.18E+04 |
| 1.25% | 0.2 mm | 1.00E+03 | 1 | 4.74E-08 | -89.2 | 72.3 | 7.55E-12 | 1.00E+12 | 2.11E+07 |
| | 0.2 mm | 1.00E+04 | 1 | 4.68E-07 | -89.6 | 149.2 | 7.45E-12 | 1.00E+12 | 2.14E+06 |
| | 0.2 mm | 5.00E+04 | 1 | 2.33E-06 | -89.7 | 211 | 7.41E-12 | 9.06E+07 | 4.30E+05 |
| | 0.2 mm | 1.00E+05 | 1 | 4.65E-06 | -89.8 | 263 | 7.40E-12 | 5.65E+07 | 2.15E+05 |
| | 0.2 mm | 5.00E+05 | 1 | 2.35E-05 | -89.6 | 155 | 7.47E-12 | 6.60E+06 | 4.26E+04 |
| | 0.2 mm | 7.50E+05 | 1 | 3.54E-05 | -89.5 | 112.8 | 7.52E-12 | 3.18E+06 | 2.82E+04 |
| | 0.2 mm | 1.00E+06 | 1 | 4.80E-05 | -89.5 | 115.4 | 7.64E-12 | 2.41E+06 | 2.08E+04 |

(continued)

| Water | Gap (mm) | FREQ (Hz) | V(V) | I(A) | p(degree) | Q | C(F) | R (0hm) | (0hm) |
|---|---|---|---|---|---|---|---|---|---|
| 2.50% | 0.2 mm | 1.00E+03 | 1 | 4.80E-08 | -89.6 | 127.3 | 7.64E-12 | 1.00E+12 | 2.08E+07 |
| | 0.2 mm | 1.00E+04 | 1 | 4.76E-07 | -89.8 | 235 | 7.57E-12 | 1.00E+12 | 2.10E+06 |
| | 0.2 mm | 5.00E+04 | 1 | 2.37E-06 | -89.8 | 275 | 7.54E-12 | 1.16E+08 | 4.22E+05 |
| | 0.2 mm | 1.00E+05 | 1 | 4.73E-D6 | -89.8 | 332 | 7.53E-12 | 7.01E+07 | 2.11E+05 |
| | 0.2 mm | 5.00E+05 | 1 | 2.39E-05 | -89.7 | 165.4 | 7.61E-12 | 6.92E+06 | 4.18E+04 |
| | 0.2 mm | 7.50E+05 | 1 | 3.61E-05 | -89.5 | 117 | 7.65E-12 | 3.24E+06 | 2.77E+04 |
| | 0.2 mm | 1.00E+06 | 1 | 4.88E-05 | -89.5 | 118.2 | 7.76E-12 | 2.42E+06 | 2.05E+04 |
| 5% | 0.2 mm | 1.00E+03 | 1 | 5.21E-08 | -89.4 | 100.8 | 8.29E-12 | 1.00E+12 | 1.92E-07 |
| | 0.2 mm | 1.00E+04 | 1 | 5.16E-07 | -89.7 | 194.5 | 8.22E-12 | 1.00E+12 | 1.94E+06 |
| | 0.2 mm | 5.00E+04 | 1 | 2.57E-06 | -89.8 | 234 | 8.17E-12 | 9.10E+07 | 3.89E+05 |
| | 0.2 mm | 1.00E+05 | 1 | 5.13E-06 | -89.8 | 274 | 8.17E-12 | 5.33E+07 | 1.95E+05 |
| | 0.2 mm | 5.00E+05 | 1 | 2.59E-05 | -89.6 | 146.8 | 8.23E-12 | 5.68E+06 | 3.87E+04 |
| | 0.2 mm | 7.50E+05 | 1 | 3.90E-05 | -89.5 | 108.4 | 8.27E-12 | 2.78E+06 | 2.57E+04 |
| | 0.2 mm | 1.00E+06 | 1 | 527E-05 | -89.5 | 112.9 | 8.38E-12 | 2.14E+06 | 1.90E+04 |
| 10% | 0.2 mm | 1.00E+03 | 1 | 5.74E-08 | -89.5 | 117.4 | 9.13E-12 | 1.00E+12 | 1.74E+07 |
| | 0.2 mm | 1.001E+04 | 1 | 5.68E-07 | -89.7 | 183.5 | 9.05E-12 | 1.00E+12 | 1.76E+06 |
| | 0.2 mm | 5.00E+04 | 1 | 2.83E-06 | -89.7 | 208 | 9.00E-12 | 7.35E+07 | 3.54E+05 |
| | 0.2 mm | 1.00E+05 | 1 | 5.64E-06 | -89.8 | 230 | 8.98E-12 | 4.08E+07 | 1.77E+05 |
| | 0.2 mm | 5.00E+05 | 1 | 2.84E-05 | -89.5 | 121 | 9.03E-12 | 4.26E+06 | 3.52E+04 |
| | 0.2 mm | 7.50E+05 | 1 | 4.27E-05 | -89.4 | 89.6 | 9.06E-12 | 2.10E+06 | 2.34E+04 |
| | 0.2 mm | 1.00E+06 | 1 | 5.76E-05 | -89.4 | 90.6 | 9.16E-12 | 1.57E+06 | 1.74E+04 |

Figure 3 shows the water in grease capacitance response as a function of frequency. The variation in capacitance is less than 3.4%. These results indicate that the capacitance of the composition is not very sensitive to changes in frequency. Figure 4 shows the capacitance measurements for water in grease for different percentages of water at different frequencies. Clearly, the measurements show a consistent result for the various frequencies. The capacitance change is stable and well measurable against the change of water percentage in the grease.

In Figure 5, the resistance versus water percentage in the grease is shown. Similar responses were received when the measurements were done at different frequencies. The electrical resistance decreases with increasing water content. Exceptional results at the 1.25% and 2.5% sample points are found, which are believed to be due to an inaccuracy of the water percentage in the sample or inaccuracy of the separation distance. With the measurement results shown above in Figure 3 to Figure 5, it can be concluded that the assumption of the equivalent electrical circuit shown in Figure 1 is rather reasonable.

Example 2

The same test method and procedures were adopted for measuring the remaining oil in grease. In this example grease samples of MT33 grease were used. A first sample was fresh grease. Second and third samples with reduced oil content were prepared by centrifuging the grease for 15 minutes and 30 minutes respectively.

The capacitance response chart of the three grease samples over seven frequencies is shown in Figure 6. The separation distance of the setup was 0.2 mm. The measurements show a general trend of capacitance increase against lessening of oil. Similar results were recorded with a 0.3 mm gap between the plates. The measurements show some spread. This is believed to be due to the nature of the centrifuging process for removing the oil, which causes it to be unevenly distributed within the grease. Two portions of the same sample may thus actually contain different amounts of oil.

The resistance response of the three grease samples over the frequency range is shown in Figure 7. A clear resistance difference can be seen between the fresh grease and the grease with reduced oil content. The grease that contains less oil has a somewhat lower electrical resistance than the fresh grease. However the differences are not very pronounced.

For further implementation of the technique into a grease sensor, the sensitivity of the various parameters to changes in grease composition is favoured. The parameter sensitivity for capacitance is defined as:

$$Sensitivity = \frac{C_{1.25\%} - C_{0\%}}{C_{0\%}}$$

Similar sensitivities are calculated for resistance and impedance. Figure 8 shows the parameter sensitivity of capacitance, resistance and impedance over the frequency range for the case of 1.25 % water content.

Figure 9 shows the same parameter sensitivities for the case of MT33 grease with respect to the same grease with a reduced oil content based on 15 minutes centrifuging. On comparing Figures 8 and 9, it can be noted that the parameter sensitivities due to changes in water content are significantly different to those due to reduction in oil content. In particular, the slope of the graphs is distinct, and can be used to identify and distinguish a change of water content from a change of oil content.

Figure 10 shows a matrix sensor 21 according to the present invention comprising a $12 \times 8$ matrix of electrodes 20 formed on an insulating substrate 22. The electrodes 20 are individually addressable via address lines 24 of a control circuit 26 having a multi-frequency alternating voltage source 28. A signal analyser 30, memory 32 and microprocessor 34 are also provided, forming an interrogation circuit 36. As shown in Figure 10, two blobs of grease G are deposited on the sensor 21. The electrodes 20 are each 1 mm square and made of copper and the substrate is a polyamide. Other materials such as PTFE for the substrate and gold for the electrodes may also be considered.

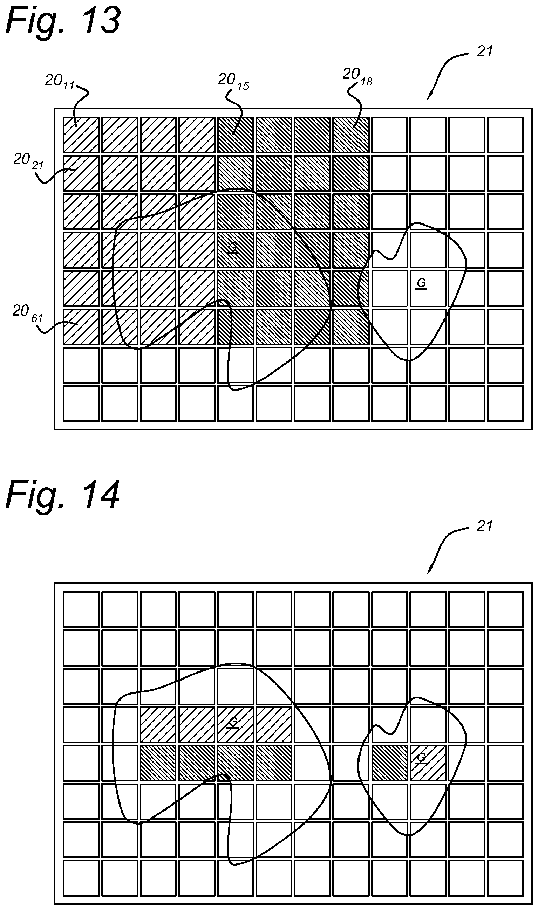

Figure 11 shows the matrix sensor 21 of Figure 10, in use in detecting the condition of the grease blobs. In a first step, the interrogation circuit 36 activates electrodes $20_{11}$ and $20_{12}$ as respective positive and negative electrodes and evaluates the current response in the signal analyser. The interrogation circuit 36 then proceeds to activate electrodes $20_{12}$, $20_{22}$, $20_{13}$ and $20_{23}$ as shown in Figure 12. Interrogation proceeds for multiple combinations of electrodes in the matrix as shown in Figure 13. An appropriate algorithm may be used to optimise the sequence of interrogations in order to determine a set of electrodes that maximises the signal representative of grease between adjacent electrodes. Such algorithms may operate in a similar manner to "battleship" strategies or may be as used for picture recognition. Figure 14 shows an optimised set of electrodes 20 registering the presence of the grease G. Further analysis of the full frequency spectrum of multi-frequency alternating voltage source 28 can then be carried out to determine the nature of the grease G based on its complex impedance, as described above in relation to Example 1.

Thus, the invention has been described by reference to the embodiment discussed above. It will be recognized that this embodiment is susceptible to various modifications and alternative forms well known to those of skill in the art. In particular, different numbers and configurations of electrodes may be provided.

**Claims**

1.   A grease condition monitoring sensor (21) comprising:

> a substrate (22) for engagement with the grease to be monitored;
> a two-dimensional matrix of electrodes (20) distributed on the substrate, the electrodes being insulated from each other and arranged for contact with the grease, such that the grease acts as a dielectric between the electrodes; and

an interrogation circuit (36) for interrogating pairs of adjacent electrodes (20) by application of an alternating voltage between the electrodes (20), measuring the current response and determining a capacitance of the respective equivalent circuits; and evaluating a composition of the grease based on the determined capacitance.

2. The sensor according to claim 1, wherein the sensor comprises at least eight electrodes, preferably more than 50 electrodes and most preferably more than 100 electrodes.

3. The sensor according to any preceding claim, wherein the interrogation circuit is connected to interrogate electrodes as either positive or negative electrodes.

4. The sensor according to claim 1 or claim 2, wherein the interrogation circuit is connected to interrogate electrodes both as positive and as negative electrodes.

5. The sensor according to any preceding claim, wherein the substrate is provided to vibrate, preferably comprising piezoelectric elements, at a frequency for the prevention of grease build-up on the electrodes.

6. The sensor according to any preceding claim, wherein the interrogation circuit comprises:

a multi-frequency alternating voltage source (28);
a control circuit (26) for applying a multi-frequency alternating voltage from the multi-frequency alternating voltage source to pairs of adjacent electrodes;
a signal analyser (30) arranged to analyse the current response to the applied voltages for determining a complex impedance of each equivalent circuit;
a memory (32) comprising preset values representative of the expected complex impedance for different conditions of the grease; and
a processor (34) for comparing the complex impedance with the preset values

7. The sensor according to any preceding claim, wherein the interrogation circuit chooses a plurality of electrodes based on the current response of their equivalent circuits and evaluates the grease condition on the basis of the plurality of electrodes.

8. The sensor according to claim 7 , wherein the plurality of electrodes is less than the totality of the electrodes in the matrix, preferably less than half of the electrodes.

9. The sensor according to any preceding claim, wherein the substrate is provided as part of a seal in a mechanical system, preferably a sealed bearing

10. A method of in-situ condition monitoring of grease within a mechanical system, the method comprising:

providing a two-dimensional matrix of electrodes (20) insulated from each other and at least partially in contact with the grease, such that the grease acts as a dielectric between at least some of the electrodes (20);
interrogating pairs of adjacent electrodes (20) by application of an alternating voltage between the electrodes (20), measuring the current response and determining a capacitance of the respective equivalent circuits; and evaluating a composition of the grease based on the determined capacitance.

11. The method according to claim 10, wherein evaluating the composition of the grease comprises comparing the capacitances with preset values to determine whether the current response is indicative of the presence of grease between the respective electrode pairs; selecting electrode pairs having a current response indicative of the presence of grease between the electrodes and evaluating the composition of the grease based on the determined capacitance of the selected electrode pairs.

12. The method according to claim 11, further comprising selecting electrode pairs that are contiguous to one another and define a single sensing region.

13. The method according to any of claims 10 to 12, wherein the electrodes comprise adjacent regions on a substrate that is in contact with the grease.

**14.** The method according to claim 10, wherein interrogation of the electrode pairs comprises application of a multi-frequency alternating voltage and furthermore determining of the complex impedance of the equivalent circuits.

**15.** The method according to claim 14, wherein the alternating voltage is applied over a spectrum of frequencies and the complex impedance is evaluated over the spectrum.

**Patentansprüche**

**1.** Fettzustandsüberwachungssensor (21), umfassend:

ein Substrat (22) zur Ineingriffnahme mit dem zu überwachenden Fett;
eine zweidimensionale Matrix aus Elektroden (20), auf dem Substrat verteilt, wobei die Elektroden voneinander getrennt und zum Kontakt mit dem Fett ausgelegt sind, so dass das Fett zwischen den Elektroden als ein Dielektrikum wirkt; und
eine Abfrageschaltung (36) zum Abfragen von Paaren von benachbarten Elektroden (20) durch Anlegen einer Wechselspannung zwischen den Elektroden (20), Messen der Stromantwort und Bestimmen einer Kapazität der jeweiligen Äquivalenzschaltungen; und Evaluieren einer Zusammensetzung des Fetts auf Basis der bestimmten Kapazität.

**2.** Sensor nach Anspruch 1, wobei der Sensor mindestens acht Elektroden umfasst, bevorzug mehr als 50 Elektroden und ganz besonders bevorzugt mehr als 100 Elektroden.

**3.** Sensor nach einem vorhergehenden Anspruch, wobei die Abfrageschaltung geschaltet ist zum Abfragen von Elektroden als entweder positiven oder negativen Elektroden.

**4.** Sensor nach Anspruch 1 oder Anspruch 2, wobei die Abfrageschaltung geschaltet ist zum Abfragen von Elektroden sowohl als positive als auch negative Elektroden.

**5.** Sensor nach einem vorhergehenden Anspruch, wobei das Substrat vorgesehen ist, bevorzugt piezoelektrische Elemente umfassend, um mit einer Frequenz für die Verhinderung von Fettaufbau auf den Elektroden zu schwingen.

**6.** Sensor nach einem vorhergehenden Anspruch, wobei die Abfrageschaltung umfasst:

eine mehrfrequente Wechselspannungsquelle (28);
eine Steuerschaltung (26) zum Anlegen einer mehrfrequenten Wechselspannung von der mehrfrequenten Wechselspannungsquelle an Paare von benachbarten Elektroden;
einen Signalanalysierer (30), der angeordnet ist zum Analysieren der Stromantwort auf die angelegten Spannungen, um eine komplexe Impedanz jeder Äquivalenzschaltung zu bestimmen;
einen Speicher (32), umfassend voreingestellte Werte, die die erwartete komplexe Impedanz für verschiedene Zustände des Fetts darstellen; und
einen Prozessor (34) zum Vergleichen der komplexen Impedanz mit den voreingestellten Werten.

**7.** Sensor nach einem vorhergehenden Anspruch, wobei die Abfrageschaltung mehrere Elektroden auf Basis der Stromantwort ihrer Äquivalenzschaltungen wählt und den Fettzustand auf Basis der mehreren Elektroden evaluiert.

**8.** Sensor nach Anspruch 7, wobei die mehreren Elektroden weniger sind als die Gesamtheit der Elektroden in der Matrix, bevorzug weniger als die Hälfte der Elektroden.

**9.** Sensor nach einem vorhergehenden Anspruch, wobei das Substrat als Teil einer Versiegelung in einem mechanischen System vorgesehen ist, bevorzugt einem abgedichteten Lager.

**10.** Verfahren zur In-situ-Zustandsüberwachung von Fett innerhalb eines mechanischen Systems, wobei das Verfahren umfasst:

Bereitstellen einer zweidimensionalen Matrix aus Elektroden (20), die voneinander getrennt sind und mit dem Fett mindestens teilweise in Kontakt stehen, so dass das Fett als ein Dielektrikum zwischen mindestens einigen der Elektroden (20) wirkt;

Abfragen von Paaren von benachbarten Elektroden (20) durch Anlegen einer Wechselspannung zwischen den Elektroden, Messen der Stromantwort und Bestimmen einer Kapazität der jeweiligen Äquivalenzschaltungen; und

Evaluieren einer Zusammensetzung des Fetts auf Basis der bestimmten Kapazität.

11. Verfahren nach Anspruch 10, wobei das Evaluieren der Zusammensetzung des Fetts das Vergleichen der Kapazitäten mit voreingestellten Werten umfasst, um zu bestimmen, ob die Stromantwort die Anwesenheit von Fett zwischen den jeweiligen Elektrodenpaaren anzeigt; Wählen von Elektrodenpaaren mit einer Stromantwort, die die Anwesenheit von Fett zwischen den Elektroden anzeigt, und Evaluieren der Zusammensetzung des Fetts auf Basis der bestimmten Kapazität der gewählten Elektrodenpaare.

12. Verfahren nach Anspruch 11, weiter umfassend das Wählen von Elektrodenpaaren, die miteinander zusammenhängen und ein einzelnes Erfassungsgebiet definieren.

13. Verfahren nach einem der Ansprüche 10 bis 12, wobei die Elektroden benachbarte Gebiete auf einem Substrat umfassen, das mit dem Fett in Kontakt steht.

14. Verfahren nach Anspruch 10, wobei eine Abfrage der Elektrodenpaare das Anlegen einer mehrfrequenten Wechselspannung und zudem das Bestimmen der komplexen Impedanz der Äquivalenzschaltungen umfasst.

15. Verfahren nach Anspruch 14, wobei die Wechselspannung über ein Spektrum von Frequenzen angelegt wird und die komplexe Impedanz über das Spektrum evaluiert wird.

## Revendications

1. Capteur (21) de surveillance d'état de graisse comprenant :

   un substrat (22) pour une interaction avec la graisse à surveiller ;
   une matrice bidimensionnelle d'électrodes (20) réparties sur le substrat, les électrodes étant isolées les unes des autres et agencées pour être en contact avec la graisse, de telle sorte que la graisse sert de diélectrique entre les électrodes ; et
   un circuit d'interrogation (36) pour interroger des paires d'électrodes (20) adjacentes en appliquant une tension alternative entre les électrodes (20), mesurer la réponse de courant et déterminer une capacité des circuits équivalents respectifs ; et évaluer une composition de la graisse sur la base de la capacité déterminée.

2. Capteur selon la revendication 1, le capteur comprenant au moins huit électrodes, préférentiellement plus de 50 électrodes et plus préférentiellement plus de 100 électrodes.

3. Capteur selon l'une quelconque des revendications précédentes, le circuit d'interrogation étant connecté pour interroger des électrodes comme des électrodes soit positives, soit négatives.

4. Capteur selon la revendication 1 ou la revendication 2, le circuit d'interrogation étant connecté pour interroger des électrodes comme des électrodes à la fois positives et négatives.

5. Capteur selon l'une quelconque des revendications précédentes, le substrat étant prévu pour vibrer, préférentiellement comprenant des éléments piézoélectriques, à une fréquence destinée à éviter la formation de graisse sur les électrodes.

6. Capteur selon l'une quelconque des revendications précédentes, le circuit d'interrogation comprenant :

   une source de tension alternative multifréquence (28);
   un circuit de commande (26) destiné à appliquer une tension alternative multifréquence provenant de la source de tension alternative multifréquence à des paires d'électrodes adjacentes ;
   un analyseur de signal (30) agencé pour analyser la réponse de courant aux tensions appliquées afin de déterminer une impédance complexe de chaque circuit équivalent ;
   une mémoire (32) comprenant des valeurs prédéfinies représentant l'impédance complexe attendue pour différents états de la graisse ; et

un processeur (34) destiné à comparer l'impédance complexe avec les valeurs prédéfinies.

7. Capteur selon l'une quelconque des revendications précédentes, le circuit d'interrogation choisissant une pluralité d'électrodes sur la base de la réponse de courant de leurs circuits équivalents et évaluant l'état de graisse sur la base de la pluralité d'électrodes.

8. Capteur selon la revendication 7, la pluralité d'électrodes étant inférieure à la totalité des électrodes dans la matrice, de préférence inférieure à la moitié des électrodes.

9. Capteur selon l'une quelconque des revendications précédentes, le substrat étant prévu comme partie d'un joint d'étanchéité dans un système mécanique, préférentiellement un roulement étanche.

10. Procédé de surveillance in-situ d'état de graisse à l'intérieur d'un système mécanique, le procédé comprenant :

la fourniture d'une matrice bidimensionnelle d'électrodes (20) isolées les unes des autres et au moins partiellement en contact avec la graisse, de telle sorte que la graisse sert de diélectrique entre au moins certaines des électrodes (20) ;
l'interrogation de paires d'électrodes (20) adjacentes en appliquant une tension alternative entre les électrodes (20), la mesure de la réponse de courant et la détermination d'une capacité des circuits équivalents respectifs ; et
l'évaluation d'une composition de la graisse sur la base de la capacité déterminée.

11. Procédé selon la revendication 10, l'évaluation de la composition de la graisse comprenant la comparaison des capacités avec des valeurs prédéterminées afin de déterminer si la réponse de courant indique la présence de graisse entre les paires d'électrodes respectives ; la sélection de paires d'électrodes ayant une réponse de courant indiquant la présence de graisse entre les électrodes et l'évaluation de la composition de la graisse sur la base de la capacité déterminée des paires d'électrodes sélectionnées.

12. Procédé selon la revendication 11, comprenant en outre la sélection de paires d'électrodes contiguës les unes aux autres et définissant une région de détection unique.

13. Procédé selon l'une quelconque des revendications 10 à 12, les électrodes comprenant des régions adjacentes sur un substrat en contact avec la graisse.

14. Procédé selon la revendication 10, l'interrogation des paires d'électrodes comprenant l'application d'une tension alternative multifréquence et aussi la détermination de l'impédance complexe des circuits équivalents.

15. Procédé selon la revendication 14, la tension alternative étant appliquée sur un spectre de fréquences et l'impédance complexe étant évaluée sur le spectre.

## Fig. 1

| Normal grease | Caused by change of oil % | Caused by change of water % | Caused by change of water % |

## Fig. 2

## Fig. 3

## Fig. 4

# *Fig. 5*

# *Fig. 6*

# Fig. 7

*Fig. 8*

*Fig. 9*

Fig. 10

## Fig. 11

## Fig. 12

## Fig. 13

## Fig. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102353703
- US 2011125475 A